# EUROPÄISCHE PATENTSCHRIFT

(11) **EP 2 912 012 B1**
(45) Veröffentlichungstag und Bekanntmachung des Hinweises auf die Patenterteilung: **14.12.2016**
(21) Anmeldenummer: 13791748.0
(22) Anmeldetag: 23.10.2013
(51) Int. Cl.: C07C 303/32, C07C 309/14

(54) **VERFAHREN ZUR HERSTELLUNG VON ZWITTERIONISCHEN MONOMEREN SOWIE DIE VERWENDUNG DIESER MONOMERE**
METHOD FOR PRODUCING ZWITTERIONIC MONOMERS AND USE OF SAID MONOMERS
PROCÉDÉ DE PRODUCTION DE MONOMÈRES ZWITTERIONIQUES AINSI QUE L'UTILISATION DE CES MONOMÈRES

(30) Priorität: 24.10.2012 DE 102012110156
(43) Veröffentlichungstag der Anmeldung: 02.09.2015
(73) Patentinhaber: Karlsruher Institut Für Technologie (KIT), 76131 Karlsruhe (DE)
(72) Erfinder: KRATZER, Domenic, 76131 Karlsruhe (DE); FRIEDMANN, Christian, 76137 Karlsruhe (DE); LAHANN, Jörg, Ann Arbor, Michigan 48105 (US)
(74) Vertreter: Fitzner, Uwe
(86) Internationale Anmeldenummer: PCT/EP2013/072159
(87) Internationale Veröffentlichungsnummer: WO 2014/064146

(56) Entgegenhaltungen:
- GAUTHIER, M. ET AL.: "Sulfobetaines zwitterionomers based on n-butyl acrylate and 2-ethoxyethyl acrylate: monomer synthesis and copolymerization behavior", JOURNAL OF POLYMER SCIENCE: PART A: POLYMER CHEMISTRY, Bd. 40, 2002, Seiten 511-523, XP002719898,

## Beschreibung

Die vorliegende Erfindung betrifft ein Verfahren zur Herstellung von zwitterionischen Monomeren und deren Verwendung.

Zwitterionische Polymerbeschichtungen sind dazu geeignet, Materialien mit ganz spezifischen Oberflächeneigenschaften zu generieren. Derart modifizierte Oberflächen wurden u.a. erfolgreich auf Antifouling-bzw. antibakterielle Eigenschaften, sowie als synthetische Zellkulturmatrizen für embryonale Stammzellen getestet. Dabei stellten sich vor allem (poly)Sulfobetain-Methacrylat-beschichtete Materialien als besonders geeignet heraus. Solche (Meth)acrylate sind kompatibel mit einer Vielzahl bekannter Polymerisationsmethoden, u.a. mit oberflächeninitiierten kontrolliert/lebend radikalischen Polymerisationen, welche zur Modifizierung von Oberflächeneigenschaften ganz besonders geeignet sind. Die Herstellung der zugrundeliegenden monomeren zwitterionischen (Meth)acrylate mit anionischer Sulfonatgruppe gestaltet sich jedoch schwierig. Bisher beschränkt sich die Synthese dieser Monomere lediglich auf eine Ringöffnungsreaktion von cyclischen Sultonen.

Die Synthese der Monomere ist aufgrund der Struktur der Sultone auf zwitterionische Sulfonate mit drei bzw. vier Methylengruppen zwischen positiver und negativer Ladung gerichtet. Ein Monomer mit zwei Methylengruppen zwischen den Ladungen ist nur in äußerst schlechten Ausbeuten zugänglich. Die Struktur der Monomere, insbesondere die Alkylkettenlänge zwischen den Ladungen, beeinflusst die Polymergeometrie der resultierenden Polymerketten erheblich und hat damit großen Einfluss auf deren Eigenschaften. Bis zum jetzigen Zeitpunkt sind keine Synthesen beschrieben worden, welche Zugang zu den (meth)acrylbasierenden Sulfonaten mit variablem Ladungsabstand - d. h. weniger als drei bzw. mehr als vier Methylengruppen zwischen positiver und negativer Ladung - liefern. Bei der Synthese entsprechender neuer Monomere erweist sich vor allen Dingen das Einführen der reaktiven (Meth)acrylgruppe sowie die starke Polarität und die damit einhergehende schlechte Löslichkeit einiger Edukte bzw. resultierender Produkte als besonderes Problem. Monomere (Meth)acryl-Sulfonate mit mehr als vier Methylengruppen zwischen positiver und negativer Ladung sind daher nicht bekannt.

Aufgrund der genannten Probleme bei der Synthese von Derivaten der zwitterionischen (Meth)acrylat-Monomere sind nur Verbindungen bekannt, die aus Ringöffnungen resultieren. Die umfangreichste Substanzbibliothek aus der Stoffklasse entsprechender Sulfonate ist in P. Köberle, A. Laschewsky, Macromolecules 1994, 27, 2165-2173 veröffentlicht. Diese Veröffentlichung beschränkt sich jedoch auf Verbindungen mit drei bzw. vier Methylengruppen zwischen positiver und negativer Ladung.

Aus Y. Terayama et al., Macromolecules 2011, 44, 104-111 ist ein spezieller Syntheseweg bekannt, welcher zu einem Methacrylat mit alternativem inneren Ladungsabstand führt. Dabei wird das *N*,*N*-Dimethylaminoethylmethacrylat mit Vinylsulfonylchlorid zu einem Monomer mit zwei Methylengruppen zwischen positiver und negativer Ladung umgesetzt. Der Nachteil dieser Synthese ist deren schlechte Ausbeutung und ihre Beschränkung auf den Ladungszustand m=2.

In J.G. Weers et al., Langmuir 1991, 7, 854-867 ist eine weitere Möglichkeit dargestellt, mit welcher Sulfobetaine mit variablen inneren Ladungsabstand hergestellt werden können. Die Synthese berücksichtigt allerdings nicht die Einführung einer (Methyl)acrylgruppe, sondern beschränkt sich auf unfunktionalisierte Sulfobetaine. Ferner wird eine Herstellung von zwitterionischen Sulfonate mit drei Methylengruppen zwischen positiver und negativer Ladung in der JP 10-087 601 A beschrieben.

Die Herstellung der zwitterionischen Monomeren SEE, SBE, SPEE und SPEB ist aus Gauthier, M. et al Journal of Polymer Science: Part A: Polymer Chemistry 2002, 40, 511-523
bekannt. Keine der beschrieben Syntheseroute beinhaltet einen Schritt, wobei zwitterionische sulfonierte N,N-alkylierten Aminoalkole mit Acrylsäure oder Methylsäure in Acrylsäure oder Methacrylsäure in Gegenwart einer weiteren Säure verestert werden.

Darüber hinaus wurde in einer Reihe von Publikationen über biologische Anwendungen zwitterionischer Polymere berichtet. Eine Auswahl zeigt die folgende Übersicht:
US 2010/0068810 A1, US 2008/0181861 A1, L. G. Villa-Diaz, H. Nandivada, J. Ding, N.C.
Nogueira-de-Souza, P. H. Krebsbach, K. S. O'Shea, J. Lahann, G. D. Smith, Nat.
Biotechnol. 2010, 28, 581-583, Z. Zhang, S. Chen, Y. Chang, S. Jiang, J. Phys. Chem. B. 2006, 110, 10799-10804, W. K. Cho, B. Kong, I. S. Choi, Langmuir, 2007, 23, 5678-5682,
H. Kitano, H. Suzuki, K. Matsuura, K. Ohno, Langmuir 2010, 26, 6767-6774, W. Feng, S.
Zhu, K. Ishihara, J. L. Brash, Langmuir 2005, 21, 5980-2987, Z. Zhang, S. Chen, Y.
Chang, S. Jiang, Biomacromolecules 2006, 7, 3311-3315.

Außerdem werden polymerisierte zwitterionische Monomere mit einer Propylgruppe zwischen positiver und negativer Ladung gemäß US 2010/0068810 A1 in der Zellkultur eingesetzt.

Zwitterionische (Meth)acrylate-Monomere mit einem Abstand von C1-C6 zwischen positiver und negativer Ladung werden gemäß US 8,183,181 B1 nach einer Invertemulsion-polymerisation bei Bohrlöchern zur Erdölförderung eingesetzt.

Aufgabe der vorliegenden Erfindung ist es, eine neue effiziente Syntheseroute zur Herstellung zwitterionischer (Meth)acrylate mit variablem Ladungsabstand bzw. daraus resultierender Polymere und Copolymere zur Verfügung zu stellen.

Diese Aufgabe wird durch ein Verfahren zur Herstellung von zwitterionischen Monomeren mit variablen Ladungsabständen vorzugsweise mit m = 2 - 100, besonders bevorzugt m = 5 - 50, ganz besonders bevorzugt mit m = 5 - 20, höchst bevorzugt mit m = 5 - 16 zwischen anionischer und kationischer Gruppe gelöst, wobei
zwitterionische sulfonierte *N*,*N-*alkylierten Aminalkohole mit Acrylsäure oder Methacrylsäure in Acrvlsäure oder Methacrylsäure in Gegenwart einer weiteren Säure verestert werden.

Index m definiert darin die Anzahl der C-Atome zwischen den beiden Halogenatomen. Zur Herstellung der zwitterionischen *N*,*N*-alkyllerten Aminalkoholen werden Halogenverbindungen mit *N*,*N*-alkylierten Aminoalkoholen zu halogenierten *N*,*N-*alkylierten Aminalkoholen umgesetzt.

Vorzugsweise werden Dihalogenverbindungen, insbesondere Dihalogenalkane eingesetzt.Zur Herstellung der zwitterionischen sulfonierten *N*,*N*-alkylierten Aminalkoholen werden die zwitterionischen *N*,*N*-alkylierten Aminalkohole unter Zusatz von Metallsulfiten sulfoniert.

In einer Ausführung werden zur Herstellung der zwitterionischen sulfonierten *N*,*N-*alkylierten Aminalkoholen Halogensulfonate mit *N*,*N*-alkylierten Aminalkohole umgesetzt.

Finaler Schritt der Synthese ist eine säurekatalysierte Veresterung dieser zwitterionischen sulfonierten Alkohole, vorzugsweise Monoalkohole mit Meth- bzw. Acrylsäure in Acrylsäure oder Methacrylsäure zu dem zwitterionischen Monomer.

Im Ergebnis wird ein Monomer der Formel I 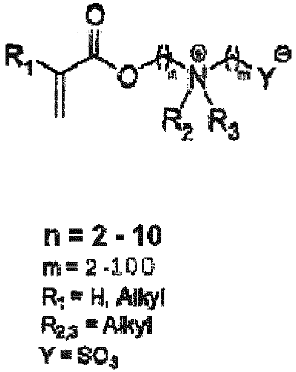 hergestellt.

Der Index n ist vorzugsweise zwischen 1-10, bevorzugt 2-8, besonderes bevorzugt 1-5, insbesondere 2, 3 oder 4. Für R₁ kommen als Alkyle vorzugsweise CH₃ (Me), CH₃CH₂ (Et) in Betracht. Die Reaktion würde aber auch mit längerkettigen Acrylsäuren funktionieren.

Der Ladungsabstand m beträgt 2-100,
7-100, 8-100, 10-100, 12-100, 10-90, 12-80, besonders bevorzugt m = 5 - 50, 7-50,8-50, 10-50. 12-50 ganz besonders bevorzugt mit m = 5 - 20,7-20, 8-20, 10-20, 12-20 höchst bevorzugt mit m = 5 - 16, 7-16, 8-16,10-16, 12-16.

Die Reste R _{2,3} können Methyl, Ethyl, Propyl, *iso*Propyl, Butyl, Pentyl sein. Alle Alkylreste können auch gemischt sein, z.B.: 1 x Methyl und 1 x Ethyl am Stickstoff oder 1 x Ethyl und 1 x Butyl.

Erfindungsgemäß können alle anorganischen Sulfite zum Einsatz kommen. Als Metallsulfite kommen vorzugsweise Alkalisulfite, insbesondere Kaliumsulfit und Natriumsulfit in Betracht. Besonders bevorzugt ist Natriumsulfit. In einer Alternative kommen Calciumsulfit, Magnesiumsulfit, Bariumsulfit, Berylliumsulfit und Übergangsmetallsulfite in Betracht.

Der in dem beschriebenen Verfahren erhaltene zwitterionische sulfonierte Alkohol wird in einem weiteren Aspekt der Erfindung in Acrylsäure unter Säurekatalyse verestert. Die Veresterung kann auch mit Methyl- oder Etylacrylsäuren durchgeführt werden. Die beschriebenen beiden Verfahrensschritte können derart geführt werden, dass durch Veresterung ein für die weitere Polymerisation geeignetes zwitterionisches Monomer entsteht. Im Ergebnis wird durch das Verfahren ein zwitterionisches Sulfonat mit endständiger Alkoholfunktionalität in der zweiten Stufe erreicht und in der dritten Stufe die finale Veresterung der Alkohole mit Acrylsäure zum fertigen Monomer erzielt:

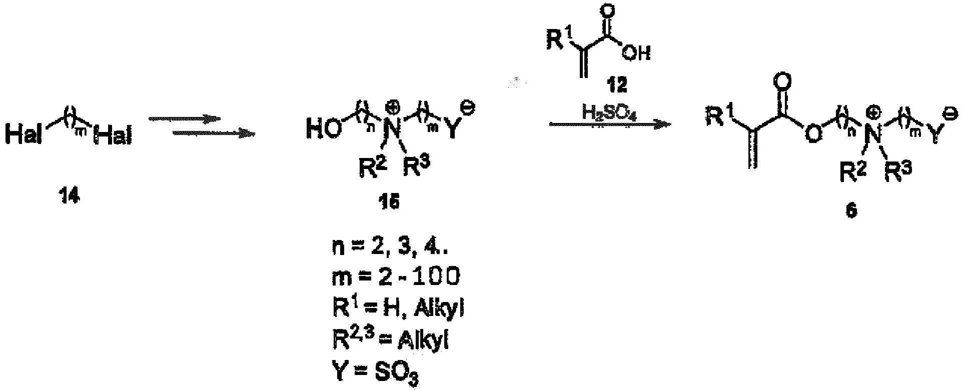

Für R₁ kommen Alkyle die oben genannte Reste zum Einsatz. Als Alkylreste kommen für R_{2, 3} ebenfalls die o. g. Reste in Betracht.

Der Ladungsabstand ist bei 2 - 100, vorzugsweise bei 5 - 50, besonders bevorzugt bei 5 - 20, ganz besonders bevorzugt bei 5 - 16.

Als Halogenverbindungen werden vorzugsweise Halogenalkane, bevorzugt Dihalogenalkane eingesetzt.

Als Halogenkomponenten HAL werden vorzugsweise Brom, Chlor, Jod oder Gemische eines oder mehrerer dieser Stoffe eingesetzt. Z. B. sind Dibromalkane, Dichloralkane, Diiodalkane von m = 5 - 20, auch entsprechende gemischte Halogenalkane, also Chlor-Bromalkane oder Brom-Iodalkane, zyklische Dihalogene wie 1,4-Dibromcyclohexan und entsprechende gemischt-halogenisierte cyclische Halogene sowie aromatische Dihalogene einsetzbar. Anstelle von Halogenen sind auch Di-Tosylate oder Di-Triflate oder Kombinationen hiervon verwendbar.
Ebenso sind Halogenen-Alkyl-Tosylate oder -Triflate oder Kombinationen hiervon verwendbar.

In dem folgenden Beispiel wird als Dihalogenalkan eine bromenthaltende Verbindung eingesetzt:

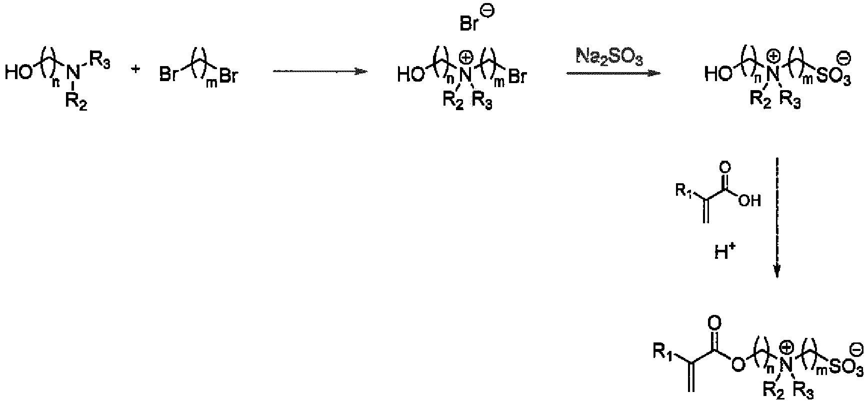

Im Ergebnis handelt es sich vorzugsweise um die Erzeugung zwitterionischer (Meth)acrylate.

Zu den bevorzugten katalytischen Säuren gehören alle Schwefelsäurederivate. Ebenso sind organische Sulfonsäuren verwendbar. D. h. neben der Schwefelsäure kommen z. B. auch para-Toluolsulfonsäuren in Betracht. Weitere Beispiele sind: Methansulfonsäure, Benzolsulfonsäure, Camphersulfonsäure, und/oder 2-(Cyclohexylamino)ethansulfonsäure.

Bei der säurekatalytischen Veresterung fungieren Carbonsäuren sowohl als Lösungsmittel als auch als Reagenz. Z. B. Lassen sich mit dem erfindungsgemäßen Verfahren nach dem folgenden Schema zwitterionische (meth)acryl-basierende Monomere herstellen. 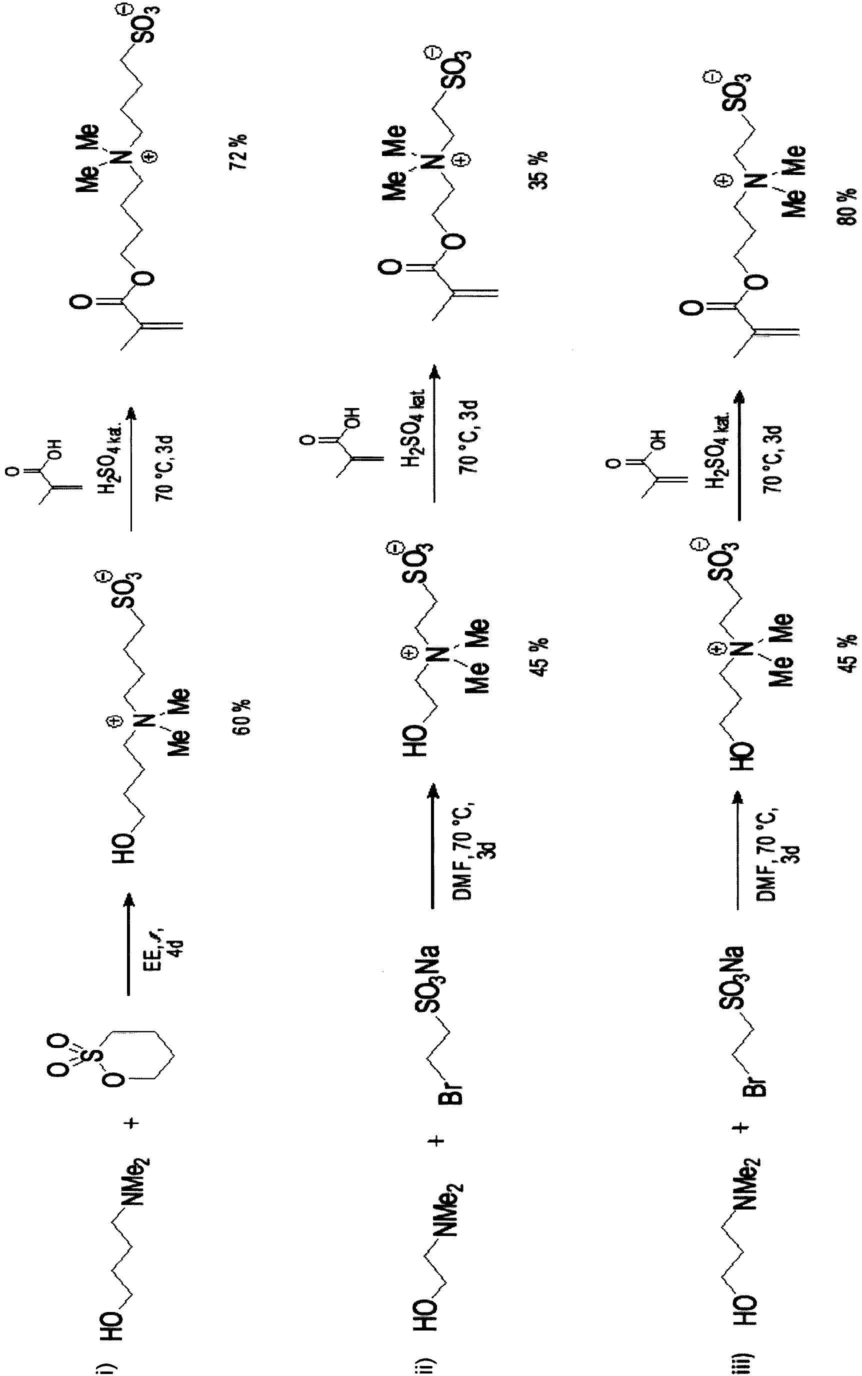 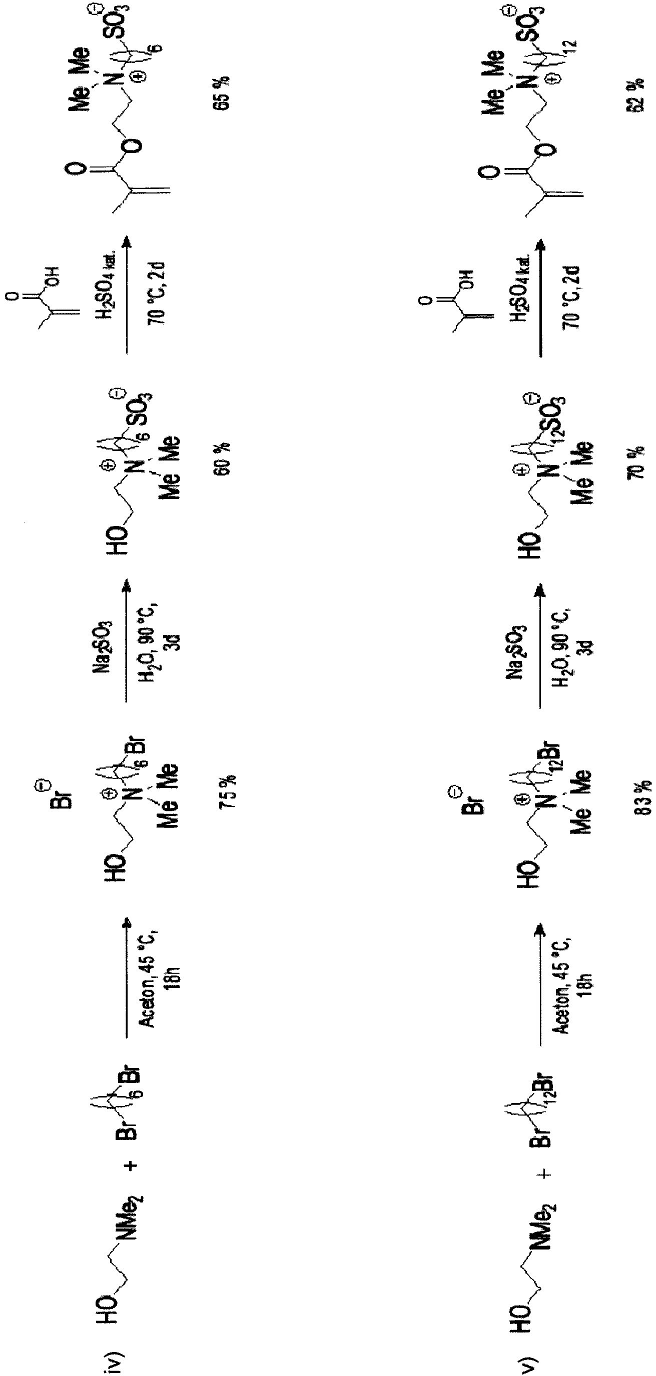 Im Folgenden sind weitere erzielbare mit dem erfindungsgemässen Verfahren erzielbare Verbindungen aufgeführt.

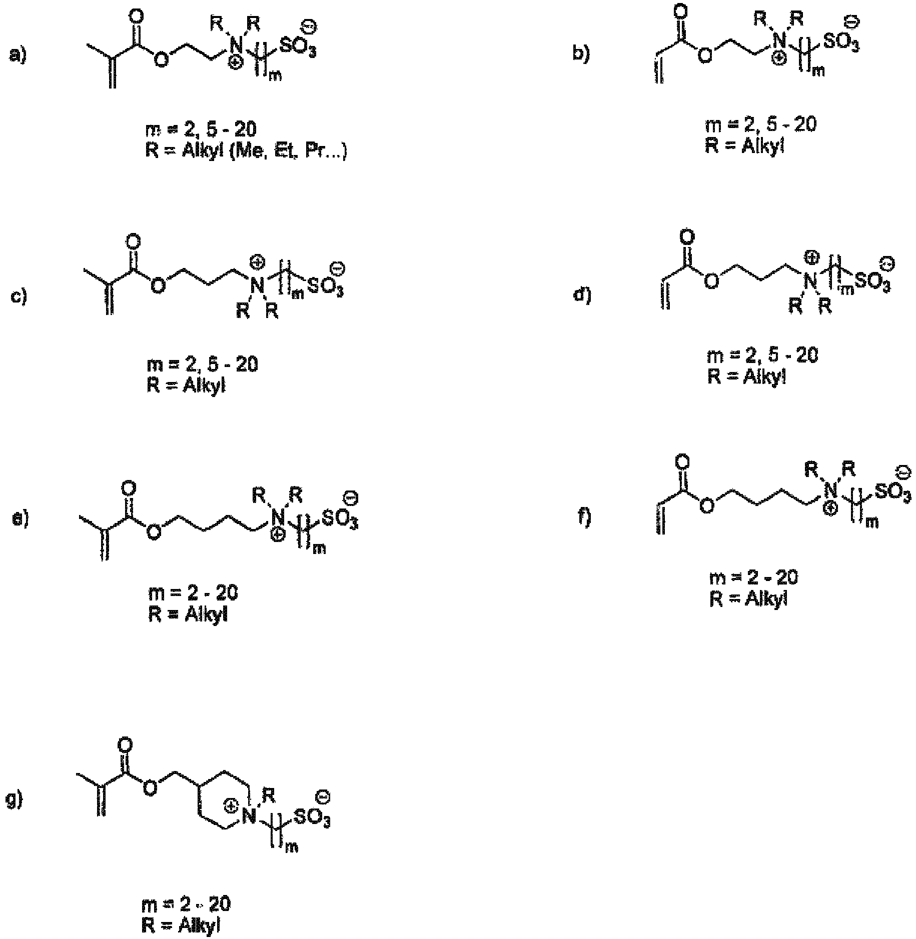

Als Alkane kommen die o. g. Reste in Betracht.

In einem weiteren Aspekt der Erfindung werden mit dem erfindungsgemäßen Verfahren hergestellte (Meth)acryl-basierende Monomere beansprucht. Es handelt sich hierbei um Monomere der allgemeinen Formel II

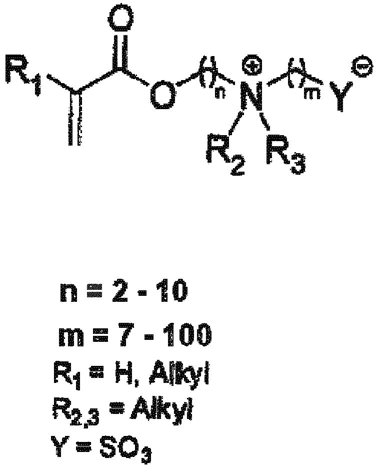

Der Index n ist vorzugsweise zwischen 1-10, bevorzugt 2-8, besonderes bevorzugt 1-5, insbesondere 2, 3 oder 4. Für R₁ kommen als Alkyle vorzugsweise CH₃ (Me), CH₃CH₂ (Et) in Betracht.

Der Ladungsabstand beträgt erfindungsgemäß -
m=7-100, 8-100, 10-100, 12-100, 10-90, 12-80, besonders bevorzugt m = 5 - 50, 7-50,8-50, 10-50. 12-50 ganz besonders bevorzugt mit m = 5 - 20,7-20, 8-20, 10-20, 12-20 höchst bevorzugt mit m = 5 - 16, 7-16, 8-16, 10-16, 12-16.

In einer weiteren Alternative der Erfindung werden auch zwitterionische Alkohole der Formel: 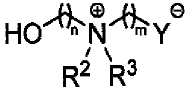 beansprucht. Die Reste haben die o.g. Bedeutung. Gleiches gilt für n und m.

Weiterhin umfasst die Erfindung in einer Alternative zwei Methacrylate der Formel

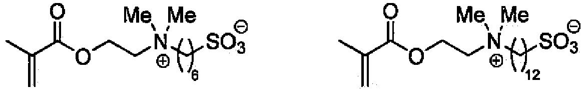

Erfindungsgemäß kann mit geringem Aufwand erreicht werden, (meth)acrylfunktionalisierte Zwitterionen herzustellen. Einsetzbar sind eine Vielzahl von Ausgangsverbindungen sowie eine Vielzahl von Variationen von Dihalogenverbindungen. Erfindungsgemäß wichtig in Zwischenstufen sind die Ammonium-Alkylsulfonate mit Alkoholfunktionalität. Durch die Einführung der (Meth)acrylgruppen können sodann polymerisierbare Produkte erhalten werden.

Aus den beschriebenen Monomeren lassen sich Polymere und Copolymere herstellen. Diese eignen sich zur Entwicklung neuer Biomaterialien. Damit werden erfindungsgemäß neue Biomaterialien mit spezifischen Eigenschaften geliefert, welche sich u. a. als synthetische Matrix für Stammzellenkulturen eignen. Die erfindungsgemäßen zwitterionischen Materialien ergeben Biomaterialien mit völlig neuen Eigenschaften. Biomaterialien sind durch ihre Biokompatibilität gekennzeichnet. Die erfindungsgemäßen zwitterionenischen Monomere und Monomere hergestellt nach dem erfindungsgemäßen Verfahren werden vorzugsweise zur Herstellung von Biomaterialien mit verbesserter Biokompatibilität verwendet.

### Beispiel

### 12-Bromo-N-(2-Hydroxyethyl)-NN-Dimethylodecan-1-Amoniumbromid

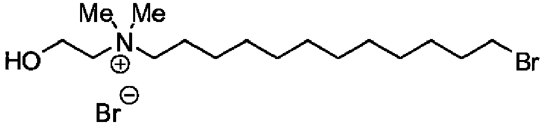

1,12 Dibromododecan (30.0 mmol, 9,84 g, 4,00 eqiv.,) wurden in 40 mm Aceton gelöst und auf 45°C erhitzt. Zu dieser Mischung wurden unter Rühren 2-(Dimethylamino)-Ethanol-7.50 mml, 0.75 ml, 1.00 eqiv.) langsam über einen Zeitraum von 6 Stunden zugegeben. Die Reaktionsmischung wurde für weitere 18 Stunden bei 45°C gerührt. Nach Abkühlen auf Raumtemperatur wurde das dunkelbraune Öl durch Dekantieren und anschließenden Filtrieren des Lösungsmittels abgetrennt. Aus der flüssigen Phase wurde Aceton durch Verdundsten abgetrennt. Der Ölrest wurde in 100 ml Ethylacetat gelöst. Aus dieser Mischung wurde das Produkt mittels Wassers extrahiert (3 x 50 ml). Die wassrigen Phasen wurden zusammengeführt und unter Verdunsten getrocknet, so dass das Produkt als braune, wachsartige Substanz vorlag.

### Ausbeute: 2.60 g (83%).

- R_{f} = 0.08 (methanol). - ¹H-NMR (500 MHz, MeOD): 4.00 -3.97 (m, 2 H, OCH₂), 3.49 - 3.38 (m, 6 H, 2 × NCH₂, BrCH₂), 3.16 (s, 6 H, 2 × NCH₃), 1.87-1.76 (m, 4 H, 2 × CH₂), 1.46-1.33 (m, 16 H, 8 × CH₂) ppm. - ¹³C-NMR (125 MHz, MeOD): 66.87 (-, CH₂), 66.55 (-, CH₂), 56.92 (-, CH₂), 52.22 (+, 2 × NCH₃), 34.50 (-, CH₂), 34.04 (-, CH₂), 30.63 (-, CH₂), 30.61 (-, 2 × CH₂), 30.56 (-, CH₂), 30.25 (-, CH₂), 29.88 (-, CH₂), 29.20 (-, CH₂), 27.45 (-, CH₂), 23.69 (-, CH₂) ppm.

### 12-[(2-Hydroxyethyl) Dimethylamonio]dodecansulfonat

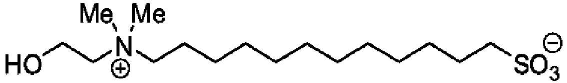

12-Bromo-N-(2-Hydroxvethyl)-NN-Dimethyldodecan-1-Amoniumbromid (4) (4.94 mmol, 2.06 g, 1.00 eqiv.) wurden in 15 ml Wasser gelöst und unter Rückfluss erhitzt. Nach 10 Minuten wurde Natriumsulfit (5.93 mmol, 747 mg, 1.20 eqiv.) zu dieser Lösung hinzugefügt. Das Reaktionsgemisch wurde dann für weitere 72 Stunden bei 90°C gerührt. Nach Abkühlen auf Raumtemperatur wurde das Lösungsmittel bei Unterdruck entfernt. Der weiße Rest wurde in 100 ml Methanol gelöst und gefiltert. Anschließend wurde das Methanol bei Unterdruck entfernt und das Rohprodukt an Silica absorbiert und einer Flash-Chromatographie (Silica, Methanol) unterworfen. So wurde das Produkt als weißer Feststoff erhalten.

### Ausbeute:1.17 g (70%).

- **R*_{f}*** = 0.23 (methanol). 0.14 (dichloromethane/methanol 2/1). - **¹****H-NMR** (500, MHz, MeOD): 4.00-3.97 (m, 2 H, OCH₂), 3.47-3.45 (m, 2 H, NCH₂), 3.41-3.38 (m, 2 H, NCH₂), 3.15 (s, 6 H, 2 x NCH₃), 2.79-2.76 (m, 2H, CH₂SO₃)_{,} 1.83-1.75 (m, 4 H, 2 × CH₂), 1.44-1.33 (m, 16 H, 8 x CH₂) ppm. - **¹³****C-NMR** (125 MHz, MeOD): 66.87 (-, CH₂), 66.52 (-, CH₂) 56.92 (-, CH₂) 52.73 - CH₂) 52.19 (+, 2 × NCH₃), 30.23 (-, 2 x CH₂), 30.20 (-, CH₂), 30.08 (-, 2 × CH₂), 30.00 (-, CH₂), 29.58 (-, CH₂),_27.34 (-, CH₂) 25.89 (-, CH₂), 23.59 (-, CH₂) ppm. - **FT-IR** (ATR): v = 3420 (w), 3298 (w), 2961 (vw). 2914 (w), 2846 (w), 1638 (vw), 1482 (w), 1463 (w), 1354 (vw), 1215 (w), 1171 (m), 1096 (m), 1072 (w), 1039 (m), 1004 (w), 986 (w), 970 (w), 924 (w), 791 (w), 601 (m), 540 (w), 521 (m), 450 (w) cm⁻¹. - **MS (FAB),** m/z (%): 338.2 (100) [M]⁺, 256.4 (11), 154.3 (9), 89.4 (10). - **HR-MS FAB** calcd for C₁₆H₃₆NO₄S: 338.2365, found 338.2368 [M]⁺.

### 12-[[2-(Methacryloyloxy)ehtyl] (Dimethyl)Amoniol-1-Dodecansulfonat (I und II)

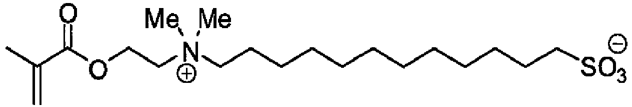

Ein ofengetrockneter, 25 ml Zweihalskolben mit Kondensor wurde evakuiert und mit Argon befüllt und anschließend mit 6 ml Metacrylsäure beladen. Nach Erhitzen auf 70°C wurde 12-[(2-Hydroxyethyl) Dimethylamonio] dodecansulfonat (5) (0.90 mmol, 304 mg) und Hydroquinon (20 mg) unter Rühren dazugegeben. Nach 30 Minuten wurden 5 Tropfen Schwefelsäure mit einer 1 ml-Spritze zu dieser Suspension hinzugefügt. Die Reaktionsmischung wurde für 72 Stunden bei 60°C gerührt. Nach Abkühlen auf Raumtemperatur wurde die flüssige Phase von dem braunen Öl durch Dekantieren getrennt. Der ölige Rest wurde anschließend in Vakuum getrocknet, während die flüssige Phase bis zur Trocknung verdunstet wurde. Nach einer Stunde wurden beide Reste in Methanol gelöst (50 ml), zusammegeführt und an Silica absorbiert. Aus der anschließenden Flash-Chromotographie (Silica, Diclomethan/Methanol 2/3) wurde das Produkt als weiter Feststoff erhalten.

Ausbeute: 226 g (62%). - **R*_{f}*** = 0.35 (methanol). - **¹H-NMR** (500 MHz, MeOD): 6.16-6.15 (m, 1H, C=CH₂), 5.74-5.72 (m, 1H, C=CH₂), 4.62-4.60 (m, 2 H, OCH₂), 3.76-3.74 (m, 2 H, NCH₂), 3.43-3.39 (m, 2 H, NCH₂), 3.17 (s, 6 H, 2 × NCH₃), 2.79-2.76 (m, 2H, CH₂SO₃), 1.97 (s, 3H, H₂C=CCH₃), 1.83-1.76 (m, 4 H, 2 × CH₂), 1.44-1.33 (m, 16 H, 2 × CH₂) ppm. - **¹³C**-**NMR** (125 MHz, MeOD): 167.69 (C_{quart}, C=O), 137.15 (C_{quart}, C=CH₂), 127.26 (-, C=CH₂), 66.66 (-, CH₂), 63.79 (-, CH₂), 59.12 (-, CH₂), 52.74 (-, CH₂), 51.92 (+, 2 × NCH₃), 30.26 (-, 2 × CH₂), 30.11 (-, 2 × CH₂), 30.08 (-, CH₂), 29.61 (-, 2 × CH₂), 27.36 (-, CH₂), 25.90 (-, CH₂), 23.61 (-, CH₂), 18.43 (+, H₂C=CCH₃) ppm. - **MS (FAB),** m/z (%): 406.1 (100) [M]⁺, 324.2 (8), 154.3 (9), 113.4 (34). - **HR-MS (FAB)** calcd for C₂₀H₄₀NO₅S: 406.2627, found 406.2629 [M]⁺.

## Patentansprüche

1. Verfahren zur Herstellung von zwitterionischen Monomeren mit variablem Ladungsabstand m zwischen anionischer und kationischer Gruppe, **dadurch gekennzeichnet, dass** zwitterionische sulfonierte *N,N*-alkylierten Aminalkohole mit Acrylsäure oder Methacrylsäure in Acrylsäure oder Methacrylsäure in Gegenwart einer weiteren Säure verestert werden.

2. Verfahren nach Anspruch 1, **dadurch gekennzeichnet, dass** zur Herstellung der zwitterionischen *N,N*-alkylierten Aminalkoholen Halogenverbindungen mit *N,N*-alkylierten Aminoalkoholen zu halogenierten *N,N*-alkylierten Aminalkoholen umgesetzt werden.

3. Verfahren nach Anspruch 2, **dadurch gekennzeichnet, dass** als Halogenverbindungen Dihalogenverbindungen eingesetzt werden.

4. Verfahren nach Anspruch 2, **dadurch gekennzeichnet, dass** als Halogenverbindungen Halogenkohlenwasserstoffe eingesetzt werden.

5. Verfahren nach Anspruch 4, **dadurch gekennzeichnet, dass** Dihalogenalkane eingesetzt werden.

6. Verfahren nach einem der Ansprüche bis 5, **dadurch gekennzeichnet, dass** anstelle von Halogenverbindungen Di-Tosylate oder Di-Triflate bzw. deren Gemische eingesetzt werden.

7. Verfahren nach Anspruch 1, **dadurch gekennzeichnet, dass** zur Herstellung der zwitterionischen sulfonierten *N,N*-alkylierten Aminalkoholen die zwitterionischen *N,N-*alkylierten Aminalkohole nach Anspruch 2 unter Zusatz von Metallsulfiten sulfoniert werden.

8. Verfahren nach Anspruch 7, **dadurch gekennzeichnet, dass** als Metallsulfit Alkalisulfite, Erdalkalisulfite oder Übergangsmatallsulfite eingesetzt werden.

9. Verfahren nach Anspruch 1, **dadurch gekennzeichnet, dass** zur Herstellung der zwitterionischen sulfonierten *N,N*-alkylierten Aminalkoholen Halogensulfonate mit *N,N-*alkylierten Aminalkohole umgesetzt werden.

10. Verfahren nach Anspruch 1, **dadurch gekennzeichnet, dass** als katalytisch wirkende Säure Schwefelsäure oder para-Toluolsulfonsäure oder deren Derivate eingesetzt werden.

11. Zwitterionische Monomere der Formel II

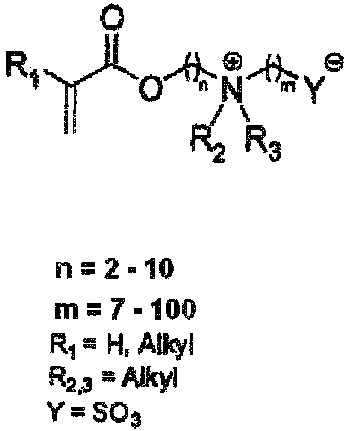

12. Zwitterionische Acrylate bzw. Methacrylate nach Anspruch 11, **dadurch gekennzeichnet**, das R_{2,3} Methyl, Ethyl, Propyl, isoPropyl, Pentyl oder Gemische eines oder mehrerer dieser Reste sind.

13. Zwitterionische Acrylate bzw. Methacrylate nach Anspruch 11 oder 12, **dadurch gekennzeichnet**, das der Ladungsabstand m = 7-50 beträgt.

14. Verwendung der Monomere nach einem der Ansprüche 11 bis 13 zur Herstellung von Copolymeren oder Polymeren oder synthetischen Matrices für Zellkulturen.

15. Verwendung der Monomere nach einem der Ansprüche 11 bis 13 zur Herstellung von synthetischen Matrices für Stammzellkulturen.

## Claims

1. A method for producing zwitterionic monomers having variable charge distance m between an anionic group and a cationic group, **characterized in that** zwitterionic sulfonated *N,N*-alkylated amino alcohols are esterified with acrylic acid or methacrylic acid in acrylic acid or methacrylic acid in the presence of a further acid.

2. The method as claimed in claim 1, **characterized in that**, to produce the zwitterionic *N*,*N-*alkylated amino alcohols, halogen compounds are reacted with *N,N*-alkylated amino alcohols to give halogenated *N,N*-alkylated amino alcohols.

3. The method as claimed in claim 2, **characterized in that** the halogen compounds used are dihalogen compounds.

4. The method as claimed in claim 2, **characterized in that** the halogen compounds used are halohydrocarbons.

5. The method as claimed in claim 4, **characterized in that** dihaloalkanes are used.

6. The method as claimed in any of claims to 5, **characterized in that** ditosylates or ditriflates or mixtures thereof are used instead of halogen compounds.

7. The method as claimed in claim 1, **characterized in that**, to produce the zwitterionic sulfonated *N,N*-alkylated amino alcohols, the zwitterionic *N,N*-alkylated amino alcohols as claimed in claim 2 are sulfonated by addition of metal sulfites.

8. The method as claimed in claim 7, **characterized in that** the metal sulfites used are alkali sulfites, alkaline earth sulfites or transition metal sulfites.

9. The method as claimed in claim 1, **characterized in that**, to produce the zwitterionic sulfonated *N,N*-alkylated amino alcohols, halosulfonates are reacted with *N,N*-alkylated amino alcohols.

10. The method as claimed in claim 1, **characterized in that** the catalytically active acids used are sulfuric acid or para-toluenesulphonic acid or derivatives thereof.

11. Zwitterionic monomers of the formula II

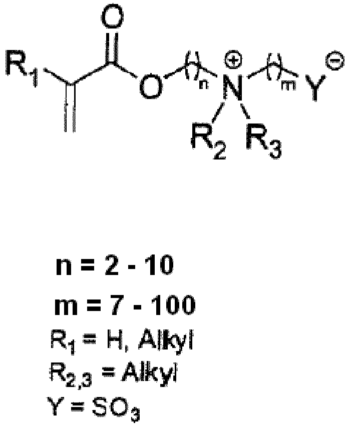

12. Zwitterionic acrylates or methacrylates as claimed in claim 11, **characterized in that** R_{2,3} are methyl, ethyl, propyl, isopropyl, pentyl or mixtures of one or more of said residues.

13. Zwitterionic acrylates or methacrylates as claimed in claim 11 or 12, **characterized in that** the charge distance is m = 7-50.

14. The use of the monomers as claimed in any of claims 11 to 13 for producing copolymers or polymers or synthetic matrices for cell cultures.

15. The use of the monomers as claimed in any of claims 11 to 13 for producing synthetic matrices for stem cell cultures.

## Revendications

1. Méthode pour produire des monomères zwitterioniques ayant une variable distance de charge m entre un groupe anionique et un groupe cationique, **caractérisée en ce que** les *N,N*-alkylés amino alcools zwitterioniques et sulfonés sont estérifiés avec de l'acide acrylique ou l'acide méthacrylique dans l'acide acrylique ou l'acide méthacrylique dans la présence d'un autre acide.

2. La méthode selon la revendication 1, **caractérisée en ce que**, pour produire les *N,N*-alkylés amino alcools zwitterioniques, des composés halogènes sont fait réagir avec des *N,N*-alkylés amino alcools pour donner des *N,N*-alkylés amino alcools halogènes.

3. La méthode selon la revendication 2 , **caractérisée en ce que** les composés halogènes usés sont des composés dihalogènes.

4. La méthode selon la revendication 2, **caractérisée en ce que** les composés halogènes usés sont des halohydrocarbones.

5. La méthode selon la revendication 4 , **caractérisée en ce que** des dihaloalcanes sont usés.

6. La méthode selon l'une des revendications jusqu'à 5, **caractérisée en ce que** des ditosylates ou des ditriflates ou des mélanges de ceux-ci sont usés au lieu des composés halogènes.

7. La méthode selon la revendication 1, **caractérisée en ce que**, pour produire des *N,N*-alkylés amino alcools zwitterioniques et sulfonés, des *N,N*-alkylés amino alcools zwitterioniques, comme revendiqués dans la revendication 2, sont sulfonés par addition des sulfites de métal.

8. La méthode selon la revendication 7, **caractérisée en ce que** des sulfites de métal usés sont des sulfites d'alcali, des sulfites alcalino terreux or des transitions des sulfites de métal.

9. La méthode selon la revendication 1, **caractérisée en ce que**, pour produire des *N,N*-alkyés amino alcools zwitterioniques et sulfonés, des halosulfonates sont fait réagir avec des *N,N-*alkylés amino alcools.

10. La méthode selon la revendication 1, **caractérisée en ce que** les acides usés que sont actifs dans une manière catalytique sont l'acide sulfurique ou l'acide para-toluènesulfonique ou les dérivés de ceux-ci.

11. Des monomères zwitterioniques de la formule Il

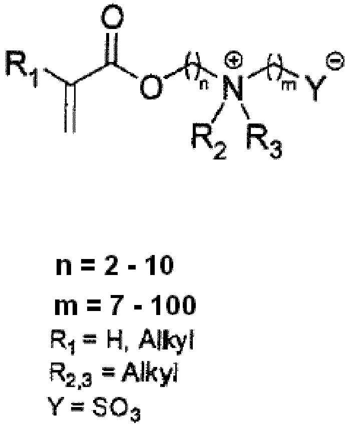

12. Des acrylates ou méthacrylates zwitterioniques selon la revendication 11, **caractérisée en ce que** R_{2,3} sont méthyle, éthyle, propyle, isopropyle, pentyle ou des mélanges de ceux-ci d'une ou de plusieurs de résidus mentionnés.

13. Des acrylates ou méthacrylates zwitterioniques selon la revendication 11 ou 12, **caractérisée en ce que** la distance de charge est m = 7-50.

14. L'usage des monomères selon les revendications 11 à 13 pour produire des copolymères ou des polymères ou des matrices synthétiques pour des cultures de cellules.

15. L' usage des monomères selon l'une des revendications 11 à 13 pour produire des matrices synthétiques pour des cultures de cellules souches.
